(19) Europäisches Patentamt
European Patent Office
Office européen des brevets

(11) Veröffentlichungsnummer : **0 145 658**
**B1**

(12) # EUROPÄISCHE PATENTSCHRIFT

(45) Veröffentlichungstag der Patentschrift : **08.07.87**

(51) Int. Cl.[4] : **C 07 C149/273**, C 07 D251/04, C 07 D257/02, C 08 K 5/37

(21) Anmeldenummer : **84810555.7**

(22) Anmeldetag : **15.11.84**

(54) **Amide von Hydroxyphenylalkylthio-alkancarbonsäuren.**

(30) Priorität : **21.11.83 US 554040**

(43) Veröffentlichungstag der Anmeldung : **19.06.85 Patentblatt 85/25**

(45) Bekanntmachung des Hinweises auf die Patenterteilung : **08.07.87 Patentblatt 87/28**

(84) Benannte Vertragsstaaten : **BE DE FR GB IT NL**

(56) Entgegenhaltungen :
**EP-A- 0 059 168**
**DE-A- 1 815 452**
**DE-A- 1 936 463**
**DE-A- 1 953 132**
**DE-A- 2 226 011**
**DE-A- 2 902 298**
**GB-A- 1 396 469**
**US-A- 3 780 103**
**US-A- 4 078 084**
**CHEMICAL ABSTRACTS, Band 86, Nr. 25, Januar 1977, Columbus, Ohio, USA, MEDVEDEV A.I. et al. "Synthesis and properties of some new derivatives of 3,5-di-tert-butyl-4-hydroxythiophenol", Seite 437, Zusammenfassung Nr. 86:5 066m**

(73) Patentinhaber : **CIBA-GEIGY AG**
**Klybeckstrasse 141**
**CH-4002 Basel (CH)**

(72) Erfinder : **Steinberg, David H.**
**2216 Wilson Avenue**
**New York New York 10469 (US)**
Erfinder : **Seltzer, Raymond**
**11 Angus Lane**
**New City New York 10956 (US)**
Erfinder : **Luzzi, John J.**
**Ann Road**
**Carmel New York 10512 (US)**
Erfinder : **Cortolano, Frank P.**
**3 East Maple Street**
**Valhalla New York 10595 (US)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 145 658 B1

**Beschreibung**

Die vorliegende Anmeldung betrifft neue Amide von Hydroxyphenyl-alkylthio-alkancarbonsäuren und deren Verwendung als Stabilisatoren für organisches Material.

Organische polymere Verbindungen wie Kunststoffe oder Harze unterliegen thermischem, oxidativem oder lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrats oder des Stabilisators ablaufen, die zu neuen Chromophoren führen. Weiterhin müssen Probleme der Verarbeitungsstabilität und der Substratverträglichkeit beachtet werden.

Ueberraschenderweise ist jetzt gefunden worden, dass die Amide von Hydroxyphenylalkylthioalkancarbonsäuren dieser Erfindung eine ungewöhnliche Kombination wünschenswerter Eigenschaften besitzen, wodurch diese Stoffe zu besonders effektiven Stabilisatoren werden. Die erfindungsgemässen Verbindungen erweisen sich als besonders nützliche Stabilisatoren für Polyolefine, schlagfestes Polystyrol, Elastomere wie Polybutadien oder Styrol-Butadien-Elastomere, bei denen der Erhalt der Elastizität und die Verhinderung von Vernetzungsreaktionen, von Versprödung, von Verfärbung, von Geruchsbildung und von Ausschwitzen des Stabilisators grundlegende Qualitätsanforderungen darstellen.

Eine Anzahl von Amiden von mindestens einer sterisch gehinderten Phenolgruppe und eine Schwefelbrücke enthaltenden Carbonsäuren ist bereits im Zusammenhang mit der Stabilisierung von organischem Material beschrieben worden, insbesondere Hydroxybenzylamide von Thioalkancarbonsäuren in US Patent 3 780 103, Hydrazide von Hydroxyphenylthio-alkancarbonsäuren in Chemical Abstracts 86, 5 066 m (1977) und Arylsulfonamide von Hydroxyphenylthio-alkancarbonsäuren in Chemical Abstracts 79, 6 089 p (1973). Aus der DE-A-2 902 298 sind 3-Hydroxybenzylverbindungen und deren Verwendung als Antioxidantien bekannt. Diese Verbindungen genügen jedoch nicht immer den heutigen Anforderungen der Technik.

Die vorliegende Erfindung betrifft Verbindungen der Formel I

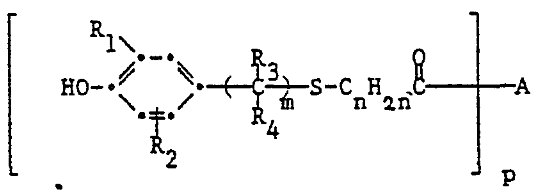

(I)

worin

p und n unabhängig voneinander eine Zahl von 1 bis 4 bedeuten, m 1 oder 2 ist,

$R_1$ und $R_2$ unabhängig voneinander $C_1$-$C_{12}$-Alkyl, $C_5$-$C_6$-Cycloalkyl, unsubstituiertes oder durch $C_1$-$C_{12}$-Alkyl substituiertes Phenyl, unsubstituiertes oder durch $C_1$-$C_{12}$-Alkyl substituiertes $C_7$-$C_9$-Aralkyl sind und $R_2$ zusätzlich Wasserstoff sein kann,

$R_3$ und $R_4$ unabhängig voneinander Wasserstoff, $C_1$-$C_{12}$-Alkyl oder Phenyl bedeuten,

A, wenn p = 1 ist, eine Gruppe —$NHR_5$, worin $R_5$ Wasserstoff, Amino, $C_1$-$C_{18}$-Alkyl oder eine Gruppe der Formel

$$-B-NH\underset{\underset{O}{\parallel}}{C}-(CH_2)_b-CH=CH_2$$

worin b eine Zahl von 0 bis 2 und B eine direkte Bindung oder $C_1$-$C_{10}$-Alkylen bedeuten,

A, wenn p = 2 ist, ein zweiwertiger Rest einer 5-7 gliedrigen heterocyclischen Verbindung mit zwei Stickstoffatomen im Ring und den freien Valenzen an den Stickstoffatomen oder eine Gruppe —HN—B—NH—, worin B die oben angegebene Bedeutung hat,

A, wenn p = 3 ist, eine Gruppe der Formel

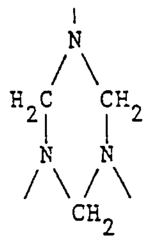

oder der Formel

$$R_6-\underset{|}{N}-(CH_2)_c-\underset{|}{N}-(CH_2)_c-\underset{|}{N}-R_6$$

worin c eine Zahl von 2 bis 6 bedeutet und $R_6$ Wasserstoff oder $C_1$-$C_4$-Alkyl ist, und A, wenn p = 4 ist, eine Gruppe der Formel

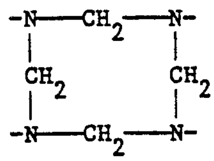

oder der Formel

$$R_6-\underset{|}{N}-(CH_2)_c-\underset{|}{N}-(CH_2)_c-\underset{|}{N}-(CH_2)_c-\underset{|}{N}-R_6$$

ist, worin c und $R_6$ die oben angegebene Bedeutung haben.

Von besonderem Interesse sind Verbindungen der Formel I, worin $R_2$ in ortho-Stellung zur Hydroxygruppe ist und Methyl oder tert.-Butyl bedeutet und $R_1$ tert.-Butyl ist. $R_3$ und $R_4$ sind bevorzugt Wasserstoff.

Bedeuten etwaige Substituenten $C_1$-$C_{12}$-Alkyl, so handelt es sich um geradkettiges oder verzweigtes Alkyl, wie z. B. Methyl, Ethyl, n-Propyl, Isopropyl, n-Butyl, sec.-Butyl, tert.-Butyl, n-Pentyl, tert.-Pentyl, n-Hexyl, 2-Ethylhexyl, n-Octyl, 1,1,3,3-Tetramethylbutyl, Decyl oder Dodecyl. $C_1$-$C_8$-Alkyl ist bevorzugt. $R_5$ hat als $C_1$-$C_{18}$-Alkyl z. B. die gleiche Bedeutung wie oben und zusätzlich beispielsweise Tridecyl, Tetradecyl, Hexadecyl und Octadecyl. $R_3$ ist bevorzugt $C_4$-$C_{12}$-Alkyl. $R_6$ ist als $C_1$-$C_4$-Alkyl z. B. Methyl, Propyl, n-Butyl und insbesondere Ethyl.

$C_5$-$C_6$-Cycloalkyl ist z. B. Cyclopentyl oder bevorzugt Cyclohexyl.

$R_1$ und $R_2$ bedeuten als Aralkyl z. B. Benzyl, α-Methylbenzyl oder α,α-Dimethylbenzyl.

Durch Alkyl substituiertes Phenyl ist beispielsweise Tolyl, Mesityl oder Xylyl.

B bedeutet als $C_1$-$C_{10}$-Alkylen z. B. Methylen, Ethylen, Propylen, Trimethylen, 2,2-Dimethylpropan-1,3-diyl, Tetramethylen, Pentamethylen, Hexamethylen, Ocatmethylen oder Decamethylen und ist bevorzugt geradkettiges $C_1$-$C_6$-Alkylen.

Wenn p = 2 ist und A den Rest einer heterocyclischen Verbindung mit zwei Stickstoffatomen bedeutet, so sind folgende Gruppen bevorzugt

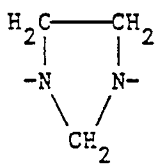 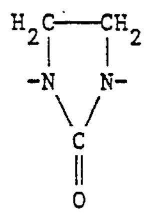 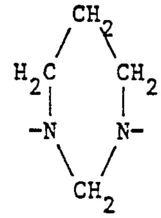 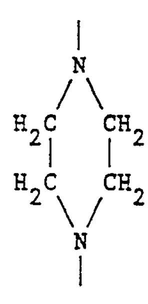

(Siehe Fortsetzung Seite 4 f.)

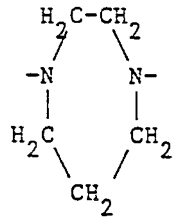

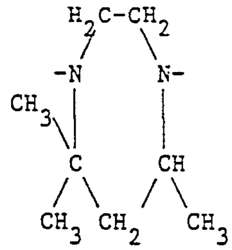

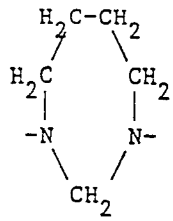

Diejenigen Verbindungen der Formel I sind bevorzugt, worin p = 2, m = 1 und n vorzugsweise 1 oder insbesondere 2 sind. Bedeutet $R_5$ eine Gruppe

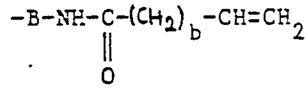

so ist es bevorzugt die Gruppe

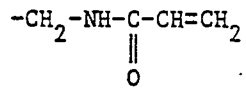

A, wenn p = 1 ist, ist bevorzugt eine Gruppe —$NHR_5$, worin $R_5$ $C_4$-$C_{12}$-Alkyl ist,

A, wenn p = 2 ist, ist bevorzugt eine Gruppe —HN—B—NH—, worin B geradkettiges $C_1$-$C_6$-Alkylen bedeutet,

A, wenn p = 3 ist, ist bevorzugt eine Gruppe

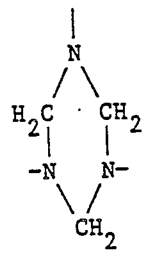

Die Verbindungen der Formel I können in Analogie zu allgemein bekannten Methoden durch Umsetzung von ca. p Mol eines Hydroxyphenylalkylmercaptans der Formel II

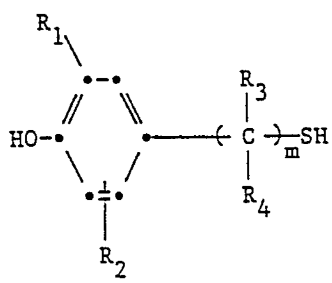

(II)

mit ca. 1 Mol eines Acrylamids der Formel III

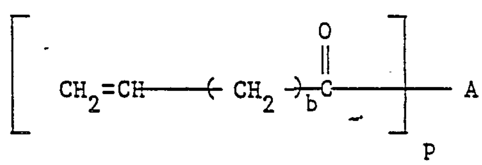

(III)

worin A, $R_1$, $R_2$, $R_3$, $R_4$, b, m und p die oben angegebene Bedeutung haben, in Gegenwart eines Protonenacceptors und vorzugsweise eines Lösungsmittels hergestellt werden.

Geeignete Protonenacceptoren sind z. B. Lithiumsalze, tertiäre Amine, Alkalimetalle, Alkalimetall- und Erdalkalimetallhydroxyde, Carbonate und ähnliche Verbindungen. Das Lösungsmittel ist vorzugsweise ein polares Lösungsmittel, wie beispielsweise Tetrahydrofuran, Dimethylformamid oder ein Alkohol. Die Reaktionstemperatur variiert üblicherweise zwischen 25 und 50 °C.

Eine weitere Herstellungsmethode für Verbindungen der Formel I ist die Umsetzung, in Analogie zu allgemein bekannten Methoden, von ca. p Mol eines Thioalkanoylhalogenids oder Thioalkanoate der Formel IV

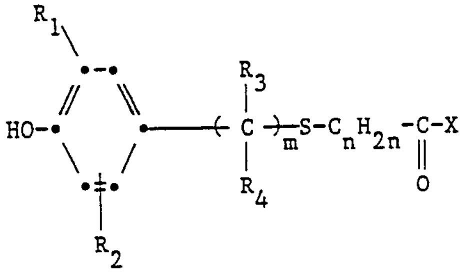

(IV)

mit ca. 1 Mol eines Amins der Formel V

$$(H)_pA \quad (V),$$

worin A, $R_1$, $R_2$, $R_3$, $R_4$, m, n und p die oben angegebene Bedeutung haben und X, Chlor, Brom oder eine Gruppe $—OR_7$ ist, worin $R_7$ Methyl oder Ethyl ist, in Gegenwart eines aromatischen Lösungsmittels und eines Protonenacceptors.

Die zur Herstellung der erfindungsgemässen Verbindungen erforderlichen Ausgangsprodukte sind bekannt und im Handel erhältlich. Sollten einige von ihnen noch neu sein, so können sie in Analogie zu bekannten Verfahren hergestellt werden.

Die erfindungsgemässen Verbindungen lassen sich als effektive Stabilisatoren für Kunststoffe, Polymere oder Harze einsetzen. Sie lassen sich auch als Stabilisatoren in Mineralölen oder synthetischen Oelen verwenden. Sie zeichnen sich insbesondere für ihre Wirksamkeit bezüglich Lichtschutz und Farbverhalten aus.

Die Erfindung betrifft daher auch eine Zusammensetzung enthaltend ein gegen oxidativen, thermischen oder strahlungsinduzierten Abbau empfindliches organisches Material und mindestens eine Verbindung der Formel I als Stabilisator, wobei als organisches Material vorzugsweise ein Polymer zu verstehen ist.

Vorzugsweise verwendet man die Verbindungen der Formel I als Stabilisatoren für Polyolefine wie beispielsweise Polyethylen oder Polypropylen ; Polystyrol, vorzugsweise schlagfestes Polystyrol ; ABS-Harz ; Elastomere wie beispielsweise Polybutandien, EPM, EPDM, SBR, Nitril-Kautschuk oder natürlicher Kautschuk.

Polymere, die mit den erfindungsgemässen Verbindungen stabilisiert werden können, sind beispielsweise :

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyisobutylen.

3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z. B. Styrol-Butadien, Styrol-Acrylnitril, Styrol-Alkylmethacrylat, Styrol-Maleinanhydrid, Styrol-Acrylnitril-Methylacrylat ; Mischungen von hoher Schlagzähigkeit aus Styrol-Copolymeren und einem anderen Polymer wie z. B. einem Polyacrylat, einem Dien-Polymeren oder einem Ethylen-Propylen-Dien-Terpolymeren ; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z. B. Styrol auf Polybutadien, Styrol und Acrylnitril auf Polybutadien, Styrol und Maleinsäure-anhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.

7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid ; sowie deren Copolymere wie Vinylchlorid-Vinylidenchlorid, Vinylchlorid-Vinylacetat oder Vinylidenchlorid-Vinylacetat.

8. Polymere, die sich von α, β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z. B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexa-methylenterephthalamid, Poly-m-phenylen-isophthalamid, sowie deren Block-Copolymere mit Polyethern wie z. B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z. B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z. B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z. B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z. B. Phthalate, Adipate, Phosphate oder Trimellithate), sowie Abmischungen synthetischer Ester mit Mineralölen in beliebigen Gewichtsverhältnissen, wie sie z. B. als Spinnpräparationen Anwendung finden, sowie deren wässrigen Emulsionen.

29. Wässrige Emulsionen natürlicher oder synthetischer Kautschuke, wie z. B. Naturkautschuk-Latex oder Latices von carboxylierten Styrol-Butadien-Copolymeren.

Die erfindungsgemässen Stabilisatoren werden den Kunststoffen in Mengen von 0,01 bis 5 Gew.-% bezogen auf das stabilisierte Material zugemischt. Je nach Verwendung und Substrat wird die Stabilisatormenge variiert. Bevorzugt verwendet man 0,05 bis 2 Gew.-% des Stabilisators, besonders bevorzugt 0,1 bis 1 Gew.-%.

Die Einarbeitung der Stabilisatorsubstanzen in die organischen Polymeren erfolgt nach bekannten Verfahren. Die Zudosierung kann während jeder Verarbeitungsstufe vor der Formgebung erfolgen. So kann der Stabilisator mit den pulverförmigen Polymeren gemischt werden, oder eine Emulsion bzw. eine Suspension des Stabilisators kann mit einer Lösung, Suspension oder Emulsion des Polymeren vermischt werden.

Die so stabilisierten Materialien können in verschiedenster Form angewendet werden, z. B. als Folien,

Fasern, Bändchen, Formmassen, Profile oder als Bindemittel für Lacke, Klebstoffe oder Kitte.

In der Praxis können die Verbindungen der Formel I zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z. B. weitere aminische Antioxidantien, Metallpassivatoren, Rostinhibitoren, Viskositäts-Index Verbesserer, Stockpunkterniedriger, Dispergiermittel/Tenside und Verschleissschutzadditive. Als weitere Additive, mit denen zusammen die erfindungsgemäss verwendeten Stabilisatoren eingesetzt werden können, sind beispielsweise zu nennen :

1. Antioxidantien

1.1. Alkylierte Monophenole
2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone
2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenylether
2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole
2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercapto-butan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

1.5 Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester

3,5-Di-tert.butyl-4-hydroxybenzyl-phosphonsäure-monoethylester, Calcium-salz.

1.6. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.8. Ester der β-(5-tert.butyl-4-hydroxy-3-methylphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.9. Amide der β-(3,5-Di-tert.butyl-4-hydroxyphenyl)-propionsäure, wie z. B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

2. UV-Absorber und Lichtschutzmittel

2.1. 2-(2'-Hydroxyphenyl)-benztriazole, wie z. B. das 5'-Methyl-, 3',5'-Di-tert.butyl-, 5'-Tert.butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z. B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z. B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z. B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

2.5. Nickelverbindungen, wie z. B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden wie n-Butyl amin, Triethanolamin oder N-Cyclohexyl-diethanolamin, Nickeldibutyldithiocarbamat, Nickelsalze von 4-Hydroxy-3, 5-di-tert.butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z. B. Bis-(2, 2, 6, 6-tetramethylpiperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin,

Tris-(2,2,6,6-tetramethyl-4-piperidyl)-nitrilotriacetat,
Tetrakis-(2,2,6,6-tetramethyl-4-piperidyl)-1,2,3,4-butantetracarbonsäure,
1,1'-(1,2-Ethandiyl)-bis-(3,3,5,5-tetramethyl-piperazinon).

2.7. Oxalsäurediamide, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdi phosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit.

5. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z. B. Kupfersalze in Kombination mit Jodiden und/oder Phosphorverbindungen und Salze des zweiwertigen Mangans.

7. Basische Co-Stabilisatoren, wie z. B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert.Butylbenzoesäure, Adipinsäure, Diphenylessigsäure.

9. Füllstoffe und Verstärkungsmittel, wie z. B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgatoren, Pigmente, Optische Aufheller, Flammschutzmittel, Antistatika, Treibmittel.

Für den Einsatz zusammen mit den erfindungsgemässen Verbindungen bevorzugte Costabilisatoren sind Antioxidantien, Lichtschutzmittel, Metalldesaktivatoren, Phosphite, Phosphonite und Thiosynergisten (peroxidzerstörende Verbindungen).

Die folgenden Beispiele erläutern die Erfindung. Alle Mengenangaben entsprechen Gewichtsteilen soweit nicht anderweitig angegeben.

Beispiel 1

N,N'-Hexamethylen-bis-[3-(3,5-di-t-butyl-4-hydroxybenzylthio)-propionamid]

In einem mit Rührer, Thermometer, Kühler und Tropftrichter versehenen Kolben werden 13,72 g (0,04 Mol) 3-(3,5-Di-tert.butyl-4-hydroxybenzylthio)-propionylchlorid in 70 ml Toluol vorgelegt. Der Kolben wird in einem Eisbad getaucht bei 5 °C und danach werden unter Stickstoff 2,32 g (0,02 Mol) 1,6-Hexandiamin, 4,05 g Triethylamin und 30 ml Toluol innerhalb 30 Minuten zugegeben. Man rührt eine Zeitlang und gibt dann weitere 50 ml Toluol hinzu um das Rühren zu erleichtern. Der erhaltene Niederschlag wird nacheinander mit Toluol und Diethylether gewaschen, von Lösungsmittelresten befreit und der Rückstand chromatographisch gereinigt. Die erhaltene glasige Substanz wird mit heissem Cyclohexan vermahlen und filtriert. Es kristallisieren weisse Kristalle aus, die umkristallisiert das erwünschte Produkt mit Smp. 126-130 °C liefern.

Analyse für $C_{42}H_{68}N_2O_4S_2$
Berechnet : C 69,2, H 9,4 S 8,8, N 3,8
Gefunden : C 69,1, H 9,3 S 8,7, N 3,6

Beispiele 2-4

Die in Tabelle I aufgelisteten Verbindungen sind gemäss der in Beispiel 1 beschriebenen Methode hergestellt worden.

Tabelle I

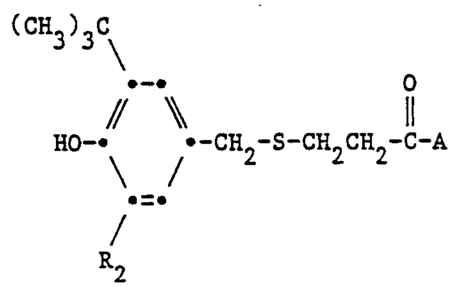

| Beispiel | $R_2$ | A | m.p. |
|---|---|---|---|
| 2 | $-C(CH_3)_3$ | $-NH-C_{12}H_{25}$ | 50-55°C |
| 3 | $-C(CH_3)_3$ | $-NH-NH_2$ | 104-107°C |
| 4 | $-CH_3$ | $-NH-NH_2$ | Syrup |

Beispiel 5

N-tert.Butyl-[3-(3-tert.-butyl-5-methyl-4-hydroxybenzylthio)propionamid]

In einem mit Rührer, Thermometer, Kühler und Tropftrichter versehenen Kolben werden unter Stickstoff 12,7 g (0,1 Mol) N-tert.-Butylacrylamid, 0,2 g Triethylamin und 100 ml Methanol vorgelegt. Das Gemisch wird auf 40 °C erwärmt und danach 22,1 g (0,105 Mol) 3-tert.-Butyl-5-methyl-4-hydroxybenzylmercaptan und 50 ml Methanol innerhalb einer Stunde zugegeben. Man lässt bei 40 °C 3 Stunden und danach 18 Stunden bei Raumtemperatur weiterreagieren.

Die Gegenwart von nicht umgesetzten Mercaptan erfordert die Zugabe von 0,1 g Benzyltrimethylfluorid als Katalysator un die weitere Reaktion bei 40 °C und bei Zimmertemperatur. Das Methanol wird dann entfernt und das Produkt chromatographisch gereinigt. Man erhält eine halbfeste Substanz.

Analyse für $C_{19}H_{31}NO_2S$
Berechnet : C 67,6, H 9,3, S 9,5, N 4,2
Gefunden : C 67,9, H 8,9, S 9,3, N 4,3

Beispiele 6-10

Die in Tabelle II aufgelisteten Verbindungen sind gemäss der in Beispiel 5 beschriebenen Methode hergestellt worden.

Tabelle II

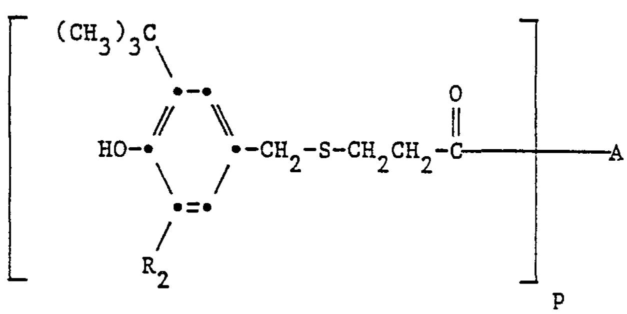

| Beispiel | p | $R_2$ | A | Smp. |
| --- | --- | --- | --- | --- |
| 6 | 1 | $-CH_3$ | $-NH_2$ | Syrup |
| 7 | 2 | $-C(CH_3)_3$ | $-HN-CH_2-NH-$ | 182-189°C |
| 8 | 2 | $-CH_3$ | $-HN-CH_2-NH-$ | 108-111°C |
| 9 | 3 | $-C(CH_3)_3$ | 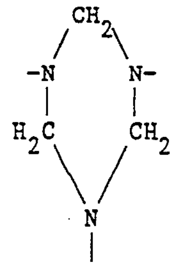 | 139-142°C |
| 10 | 3 | $-CH_3$ | 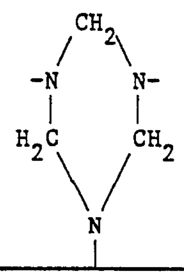 | 160-161°C |

Beispiel 11

N-Dodecyl-(3,5-di-tert.-butyl-4-hydroxybenzylthio)-acetamid

In einem mit Rührer, Thermometer, Kühler und Tropftrichter versehenen Kolben werden unter Stickstoff 18,54 g (0,1 Mol), n-Dodecylamin und 9,21 g (0,1 Mol) Thioglykolsäure vorgelegt. Man lässt die erhaltene Aufschlämmung 10 Stunden bei 140 °C reagieren. Danach werden 26,35 g (0,1 Mol) 2,6-Di-tert.-butyl-4-dimethylaminomethylphenol und 100 ml Isopropanol zum inzwischen in-situ gebildeten N-Dodecyl-β-mercapto-acetamid zugefügt. Es wird weitere 10 Stunden unter Rückfluss (80 °C) reagieren gelassen. Das Isopropanol wird dann abgetrennt und das Produkt chromatographisch isoliert. Nach dem Abtrennen des Lösungsmittels im Vakuum bleiben weisse Kristalle mit Smp. 43-46 °C zurück.

Analyse für $C_{29}H_{51}NO_2S$
Berechnet : C 72,9, H 10,8, S 6,7, N 2,9
Gefunden : C 73,0, H 10,8, S 6,7, N 3,0

Beispiel 12

Stabilisierung von schlagfestem Polystyrol

Man stellt eine Lösung von 8 Gew.-% Polybutadien-Kautschuk (Fireston DIENE 55®) in monomerem Styrol her, indem man beide Komponenten in einer Quetschmühle mischt. Gleichzeitig werden in diesem Arbeitsgang 0,1 Gew.-% des zu testenden Stabilisators zu der Lösung gegeben. Schliesslich fügt man noch 500 ppm Zn-Stearat hinzu, um später die polymerisierte Probe leicht aus dem Reaktionsgefäss ablösen zu können. Die Polymerisation wird in einem Gefäss durchgeführt, dass mit einem Wendelrührer ausgerüstet ist. Da die meisten IPS Blockpolymerisationen thermisch initiiert werden, wird in der vorliegenden Laboratoriumversion kein Starter zugesetzt. Die Reaktion wird unter Stickstoff und unter Erwärmen durchgeführt. Der Reaktor wird innerhalb einer halben Stunde auf 121 °C erwärmt, und diese Temperatur wird aufrecht erhalten, bis zwischen 30 und 35 % des Monomeren umgesetzt sind (Zeitdauer ca. 2,5 Stunden). Während der Polymerisation wird heftig gerührt, und die Rührgeschwindigkeit wird so eingestellt, dass Polymerpartikel von 2 bis 4 µm Grösse entstehen.

Anschliessend werden die Reaktionsgefässe dem Reaktor entnommen, im Stickstoffstrom geöffnet und in ein Wirbelschichtsandbad gestellt, um die Polymerisation zu vervollständigen. Die Gefässe werden

im Sandbad folgendermassen erhitzt : eine Stunde bei 100 °C um die Anfangstemperatur vorzugeben, eine weitere Stunde, um 140 °C zu erreichen und anschliessend weitere 8 Stunden, wobei die Temperatur jede Stunde um 10 °C erhöht wird, bis schliesslich ein Maximum von 220 °C erreicht ist. Nach dem Abkühlen werden die Polymerisationsgefässe zerbrochen und das Glas entfernt. Ein einziger Polymerblock wiegt im Mittel etwas über 600 g. Der Block wird in einem Vakuumofen bei 200 °C für 45 Minuten bei einem Druck von 1,33 mbar belassen, um flüchtige Bestandteile zu entfernen. Nach dieser Behandlung wird der Block sofort in einer heizbaren hydraulischen Presse bei 205 °C zwischen zwei Aluminiumfolien zu einem dicken Stab gepresst (drei Minuten Erhitzen, fünf Minuten ungeheizt).

Der Stab wird mittels einer Bandsäge zerkleinert und anschliessend granuliert. Alle Polymermischungen werden bei 205 °C extrudiert und dann zerkleinert. Die Pellets werden unter Druck bei 205 °C in dehnbare 3,2 mm dicke Streifen verformt. Diese Probestreifen werden dann bei 150 °C in einem Umluftofen gealtert, wobei sie auf Glasplatten, die sich wiederum auf rotierenden Trägern befinden, aufgebracht sind. Nach bestimmten Zeitintervallen wird der Yellowness Index der Proben gemäss ASTM D-1925-63T sowie die Dehnbarkeit bestimmt (Instron Tensile Testing Apparatus, Instron Engineering Corporation, Massachusetts) ; Ziehgeschwindigkeit : 5 mm/Minute, ASTM D-638).

In den folgenden Tabellen 1 und 2 sind die Ergebnisse der Dehnbarkeitsmessung und der Messungen des Yellowness-Index von bei 80 °C bzw. 150 °C ofengealterten Proben angegeben.

Tabelle 1

Dehnbarkeitsmessungen und Yellowness Index von bei 80 °C ofengealterten Proben mit Stabilisator

| Additiv (Produkt von Beispiel) | Stabilisatormenge (Gew.-%) | Dehnbarkeit der Probe (%) und Dauer der Hitzebelastung (h) | | | |
|---|---|---|---|---|---|
| | | 0h | 300h | 600h | 900h |
| keines | - | 33 | 9 | 3 | 3 |
| 6 | 0,1 | 47 | 36 | 29 | 24 |
| 7 | 0,1 | 58 | 35 | 31 | 28 |
| 8 | 0,1 | 56 | 38 | 35 | 29 |
| 10 | 0,1 | 51 | 36 | 28 | 30 |
| 7+DLTDP* | 0,05/0,05 | 64 | 35 | 19 | 11 |

| Additiv (Produkt von Beispiel) | Stabilisatormenge (Gew.-%) | Yellowness Index und Dauer der Hitzebehandlung (h) | | | |
|---|---|---|---|---|---|
| | | 0h | 300h | 600h | 900h |
| keines | - | 7 | 14 | 45 | 59 |
| 6 | 0,1 | -11 | -5 | 2 | 13 |
| 7 | 0,1 | -11 | -3 | -2 | 8 |
| 8 | 0,1 | -11 | -4 | 2 | 5 |
| 10 | 0,1 | -11 | -2 | 6 | 16 |
| 7+DLTDP* | 0,05/0,05 | -3 | 4 | 15 | 39 |

Tabelle 2

Dehnbarkeitsmessungen und Yellowness Index von bei 150 °C ofengealterten Proben mit Stabilisator

| Additiv (Produkt von Beispiel) | Stabilisatormenge (Gew.-%) | Dehnbarkeit der Probe (%) und Dauer der Hitzebelastung (h) | | | | |
|---|---|---|---|---|---|---|
| | | 0h | 0,5h | 1h | 1,5h | 2h |
| keines | - | 33 | 7 | 7 | 3 | 3 |
| 6 | 0,1 | 47 | 25 | 24 | 26 | - |
| 7 | 0,1 | 58 | 44 | 32 | 37 | 28 |
| 8 | 0,1 | 56 | 32 | 50 | 31 | 33 |
| 10 | 0,1 | 51 | 40 | 36 | 35 | - |
| 7+DLTDP* | 0,05/0,05 | 64 | 56 | 36 | 45 | 45 |

| Additiv (Produkt von Beispiel) | Stabilisatormenge (Gew.-%) | Yellowness Index und Dauer der Hitzebehandlung (h) | | | | |
|---|---|---|---|---|---|---|
| | | 0h | 0,5h | 1h | 1,5h | 2h |
| keines | - | 7 | 18 | 30 | 38 | 43 |
| 6 | 0,1 | -11 | -9 | -8 | -8 | -7 |
| 7 | 0,1 | -11 | -9 | -8 | -8 | -6 |
| 8 | 0,1 | -11 | -9 | -7 | -7 | -4 |
| 10 | 0,1 | -11 | -8 | -8 | -3 | - |
| 7+DLTDP* | 0,05/0,05 | -3 | -1 | 0 | 2 | 2 |

*Dilaurylthiodipropionat

Beispiel 13

Stabilisierung von ABS

Folgendes Acrylnitril-Butadien-Styrol-Latex wird durch Zugabe der Emulsionskomponente zum ABS-Latex und gründliches Vermischen hergestellt :

| | Teile |
|---|---|
| ABS Latex (Polybutadiengehalt 40 Gew.-%) | 625 |
| Verbindung von Beispiel 2 | 5 |
| Toluol | 40 |
| Alkyl-aryl-polyether-alkohol (®Triton X-100) | 4 |
| Wasser | 150 |

Ein vergleichbares Latex wird ohne Stabilisator hergestellt.

In beiden Fällen wird das erhaltene Latex einer 15 Gew. %igen wässrigen Natriumsulfatlösung bei 90 °C zugegeben um das System zu koagulieren. Das Gemisch wird filtriert und die Festsubstanz fünf Mal mit 90 °C warmem Wasser gewaschen. Vom feuchten Filtergut wird die Oxidationsbeständigkeit mit Hilfe der Differentialthermoanalyse bestimmt. Als Mass der Oxidationsbeständigkeit gilt die Temperatur $T_{max}$, bei der die exotherme Oxidationsreaktion erfolgt. Je höher der Wert, umso besser die thermische Beständigkeit. Die Resultate sind wie folgt

| | DTA (Dynamisches Verfahren) $T_{max}$ (°C) |
|---|---|
| Ohne Stabilisator | 160 |
| Mit Stabilisator (Beispiel 2) | 215 |

**Patentansprüche**

1. Verbindungen der Formel I

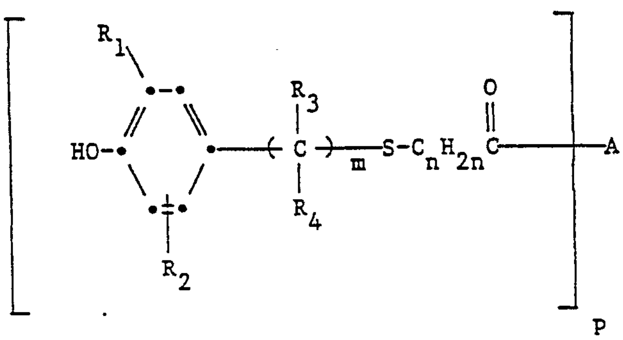

(I)

worin

p und n unabhängig voneinander eine Zahl von 1 bis 4 bedeuten,

m 1 oder 2 ist,

$R_1$ und $R_2$ unabhängig voneinander $C_1$-$C_{12}$-Alkyl, $C_5$-$C_6$-Cycloalkyl,

unsubstituiertes oder durch $C_1$-$C_{12}$-Alkyl substituiertes Phenyl, unsubstituiertes oder durch $C_1$-$C_{12}$-Alkyl substituiertes $C_7$-$C_9$-Aralkyl sind und $R_2$ zusätzlich Wasserstoff sein kann,

$R_3$ und $R_4$ unabhängig voneinander Wasserstoff, $C_1$-$C_{12}$-Alkyl oder Phenyl bedeuten,

A, wenn p = 1 ist, eine Gruppe —$NHR_5$, worin $R_5$ Wasserstoff, Amino, $C_1$-$C_{18}$-Alkyl oder eine Gruppe der Formel

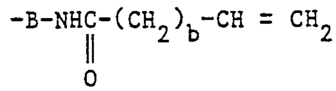

worin b eine Zahl von 0 bis 2 und B eine direkte Bindung oder $C_1$-$C_{10}$-Alkylen bedeuten,

A, wenn p = 2 ist, ein zweiwertiger Rest einer 5-7 gliedrigen heterocyclischen Verbindung mit zwei Stickstoffatomen im Ring und den freien Valenzen an den Stickstoffatomen oder eine Gruppe —HN—B—NH—, worin B die oben angegebene Bedeutung hat,

A, wenn p = 3 ist, eine Gruppe der Formel

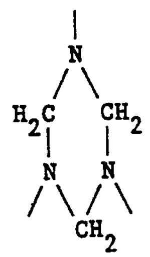

oder der Formel

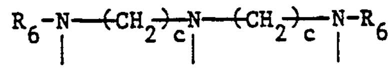

worin c eine Zahl von 2 bis 6 bedeutet und $R_6$ Wasserstoff oder $C_1$-$C_4$-Alkyl ist, und
A, wenn p = 4 ist, eine Gruppe der Formel

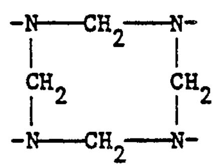

oder der Formel

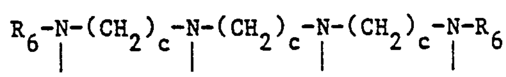

ist, worin c und $R_6$ die oben angegebene Bedeutung haben.

2. Verbindungen gemäss Anspruch 1, dadurch gekennzeichnet, dass $R_2$ in ortho-Stellung zur Hydroxy-Gruppe steht.

3. Verbindungen gemäss Anspruch 2, dadurch gekennzeichnet, dass $R_1$ tert.-Butyl und $R_2$ Methyl oder tert.-Butyl bedeuten.

4. Verbindungen gemäss Anspruch 1, dadurch gekennzeichnet, dass p = 2 ist.

5. Verbindungen gemäss Anspruch 1, dadurch gekennzeichnet, dass m = 1 ist.

6. Verbindungen gemäss Anspruch 1, dadurch gekennzeichnet, dass n = 1 oder 2 ist.

7. Verbindungen gemäss Anspruch 1, dadurch gekennzeichnet, dass n = 2 ist.

8. Verbindungen gemäss Anspruch 1, dadurch gekennzeichnet, dass $R_5$ eine Gruppe —$CH_2$—NH—C(O)—CH=$CH_2$ bedeutet.

9. Verbindungen gemäss Anspruch 1, dadurch gekennzeichnet, dass p = 1 ist und A eine Gruppe —$NHR_5$ bedeutet, worin $R_5$ $C_4$-$C_{12}$-Alkyl ist.

10. Verbindungen gemäss Anspruch 1, dadurch gekennzeichnet, dass p = 2 ist und A eine Gruppe —HN—B—NH— bedeutet, worin B geradkettiges $C_1$-$C_6$-Alkylen ist.

11. Verbindungen gemäss Anspruch 1, dadurch gekennzeichnet, dass p = 3 ist und A eine Gruppe der Formel

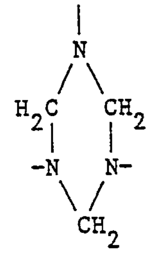

bedeutet.

12. Stoffzusammensetzung enthaltend ein gegen oxidativen, thermischen oder strahlungsinduzierten Abbau empfindliches organisches Material und mindestens eine Verbindung der Formel I gemäss Anspruch 1 als Stabilisator.

13. Stoffzusammensetzung gemäss Anspruch 12, worin unter dem organischen Material ein synthetisches Polymer zu verstehen ist.

14. Stoffzusammensetzung gemäss Anspruch 13, worin unter dem synthetischen Polymer ein Polyolefin, schlagfestes Polystyrol, ABS-Harz, Polybutadien, EPM, EPDM, SBR, Nitrilkautschuk oder natürlicher Kautschuk zu verstehen ist.

15. Stoffzusammensetzung gemäss Anspruch 12, enthaltend zusätzlich mindestens einen Costabilisator ausgewählt aus der Gruppe bestehend aus Antioxidantien, Lichtschutzmittel, Metalldesaktivatoren, Phosphite, Phosphonite und Thiosynergisten.

**Claims**

1. A compound of formula I

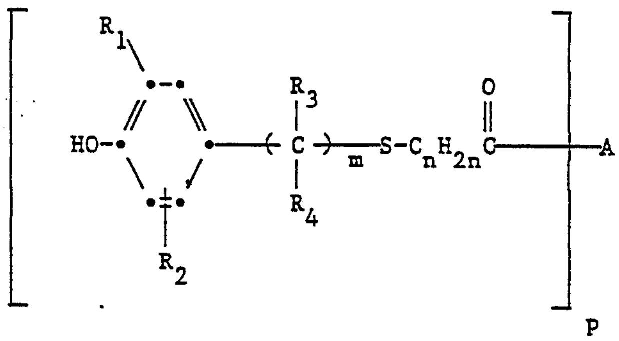

(I)

wherein

p and n are each independently 1 to 4 ;

m is 1 or 2 ;

$R_1$ and $R_2$ are each independently of the other $C_1$-$C_{12}$alkyl, $C_5$-$C_6$cycloalkyl, phenyl or phenyl substituted by $C_1$-$C_{12}$alkyl, $C_7$-$C_9$aralkyl or said aralkyl substituted by $C_1$-$C_{12}$alkyl, and $R_2$ can additionally be hydrogen ; $R_3$ and $R_4$ are each independently of the other hydrogen, $C_1$-$C_{12}$alkyl or phenyl ;

A, when p = 1, is a group —$NHR_5$, wherein $R_5$ is hydrogen, amino, $C_1$-$C_{18}$alkyl or a group of formula

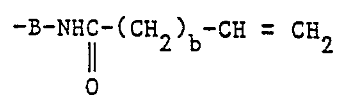

wherein b is 0 to 2 and B is a direct bond or $C_1$-$C_{10}$alkylene ;

A, when p = 2, is a bivalent radical of a 5-7 membered heterocyclic compound containing two nitrogen atoms in the ring, with the free valencies at the two nitrogen atoms, or is a group —HN—B—NH—, wherein B has the meaning given above ;

A, when p = 3, is a group of formula

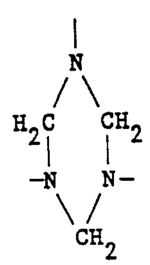

or of formula

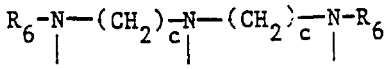

wherein c is 2 to 6 and $R_6$ is $C_1$-$C_4$alkyl ; and
A, when p = 4, is a group of formula

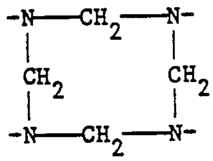

or of formula

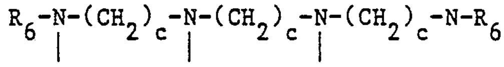

wherein c and $R_6$ have the meanings given above.

2. A compound according to claim 1, wherein $R_2$ is in the ortho position to the hydroxy group.

3. A compound according to claim 2, wherein $R_1$ is tert-butyl and $R_2$ is methyl or tert-butyl.

4. A compound according to claim 1, wherein p is 2.

5. A compound according to claim 1, wherein m is 1.

6. A compound according to claim 1, wherein n is 1 or 2.

7. A compound according to claim 6, wherein n is 2.

8. A compound according to claim 1, wherein $R_3$ is —$CH_2$—NH—C(O)—CH=$CH_2$.

9. A compound according to claim 1, wherein p is 1 and A is —$NHR_3$, wherein $R_3$ is $C_4$-$C_{12}$alkyl.

10. A compound according to claim 1, wherein p is 2 and A is —HN—B—NH—, wherein B is straight-chain $C_1$-$C_6$alkylene.

11. A compound according to claim 1, wherein p is 3 and A is a group of formula

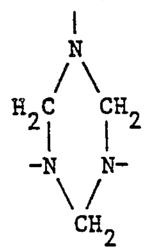

12. A composition of matter comprising an organic material subject to oxidative, thermal and actinic degradation and at least one compound of formula I according to claim 1 as stabiliser.

13. A composition according to claim 12, wherein the organic material is a synthetic polymer.

14. A composition according to claim 13, wherein the synthetic polymer is selected from the group consisting of polyolefins, impact polystyrene, ABS resin, polybutadiene, EPM, EPDM, SBR, nitrile rubber and natural rubber.

15. A composition of matter according to claim 12, which additionally contains at least one co-stabiliser selected from the group consisting of antioxidants, light stabilisers, metal deactivators, phosphites, phosphonites and thiosynergists.

**Revendications**

1. Composés répondant à la formule I :

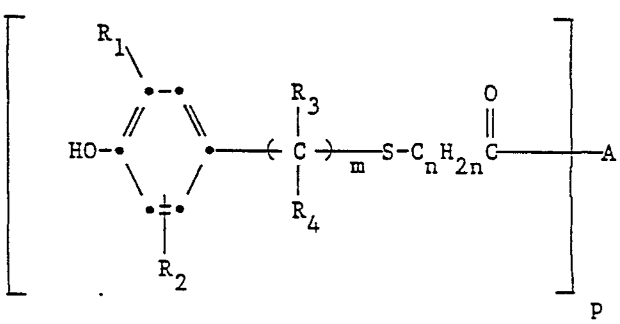

(I)

dans laquelle

p et n représentent chacun, indépendamment l'un de l'autre, un nombre de 1 à 4,

m est égal à 1 ou à 2,

$R_1$ et $R_2$ représentent chacun, indépendamment l'un de l'autre, un alkyle en $C_1$-$C_{12}$, un cycloalkyle en $C_5$ ou $C_6$, un phényle non substitué ou porteur d'un alkyle en $C_1$-$C_{12}$, ou un aralkyle en $C_7$-$C_9$ non substitué ou porteur d'un alkyle en $C_1$-$C_{12}$, et $R_2$ peut en outre représenter l'hydrogène,

$R_3$ et $R_4$ représentent chacun, indépendamment l'un de l'autre, l'hydrogène, un alkyle en $C_1$-$C_{12}$ ou un phényle,

et

A représente :

lorsque p est égal à 1, un radical —$NHR_5$ dans lequel $R_5$ représente l'hydrogène, un amino, un alkyle en $C_1$-$C_{18}$ ou un radical de formule :

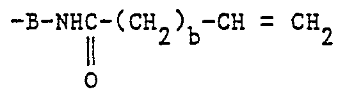

dans lequel b désigne un nombre de 0 à 2 et B une liaison directe ou un alkylène en $C_1$-$C_{10}$,

lorsque p est égal à 2, un radical bivalent d'un composé hétérocyclique dont le cycle comporte de 5 à 7 maillons et contient deux atomes d'azote, les valences libres de ce radical partant des atomes d'azote, ou un radical —HN—B—NH— dans lequel B a la signification indiquée ci-dessus,

lorsque p est égal à 3, un radical de formule :

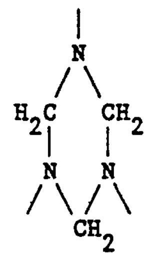

ou un radical de formule :

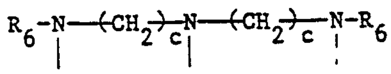

dans lequel c désigne un nombre de 2 à 6 et $R_6$ l'hydrogène ou un alkyle en $C_1$-$C_4$, et

lorsque p est égal à 4, un radical de formule :

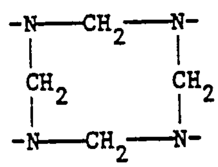

ou un radical de formule :

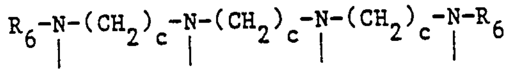

dans lequel c et $R_6$ ont les significations indiquées ci-dessus.

2. Composés selon la revendication 1 caractérisés en ce que $R_2$ est en position ortho relativement au radical hydroxy.

3. Composés selon la revendication 2 caractérisés en ce que $R_1$ est un radical tert-butyle et $R_2$ un radical méthyle ou tert-butyle.

4. Composés selon la revendication 1 caractérisés en ce que p est égal à 2.

5. Composés selon la revendication 1 caractérisés en ce que m est égal à 1.

6. Composés selon la revendication 1 caractérisés en ce que n est égal à 1 ou à 2.

7. Composés selon la revendication 1 caractérisés en ce que n est égal à 2.

8. Composés selon la revendication 1 caractérisés en ce que $R_5$ représente un radical —$CH_2$—NH—C(O)—CH=$CH_2$.

9. Composés selon la revendication 1 caractérisés en ce que p est égal à 1 et A représente un radical —$NHR_5$ dans lequel $R_5$ représente un alkyle en $C_4$-$C_{12}$.

10. Composés selon la revendication 1 caractérisés en ce que p est égal à 2 et A représente un radical —NH—B—NH— dans lequel B désigne un alkylène linéaire en $C_1$-$C_6$.

11. Composés selon la revendication 1 caractérisés en ce que p est égal à 3 et A représente un radical de formule :

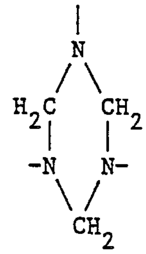

12. Composition contenant une matière organique sensible à la dégradation sous l'effet de l'oxydation, de la chaleur ou d'un rayonnement, et au moins un composé de formule I selon la revendication 1 comme stabilisant.

13. Composition selon la revendication 12 dans laquelle la matière organique est un polymère synthétique.

14. Composition selon la revendication 13 dans laquelle le polymère synthétique est une polyoléfine, un polystyrène haute résilience, la résine ABS, le polybutadiène, l'EPM, l'EPDM, le SBR, le caoutchouc nitrile ou le caoutchouc naturel.

15. Composition selon la revendication 12 qui contient en outre un co-stabilisant pris dans l'ensemble constitué par les anti-oxydants, les stabilisants à la lumière, les désactivants de métaux, les phosphites, les phosphonites et les agents de synergie sulfurés.